# EUROPÄISCHE PATENTSCHRIFT

(11) **EP 0 428 038 B1**
(45) Veröffentlichungstag und Bekanntmachung des Hinweises auf die Patenterteilung: **20.07.1994**
(21) Anmeldenummer: 90121170.6
(22) Anmeldetag: 06.11.1990
(51) Int. Cl.: A61L 15/16, A61K 9/70

(54) **Transdermale Applikation von 2-Amino-6-n-propylamino-4,5,6,7-tetrahydrobenzothiazol**
Transdermal application of 2-amino-6-n-propylamino-4,5,6,7-tetrahydrobenzothiazol
Administration transdermique de 2-amino-6-n-propylamino-4,5,6,7-tétrahydrobenzothiazol

(30) Priorität: 09.11.1989 DE 3937271
(43) Veröffentlichungstag der Anmeldung: 22.05.1991
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Zierenberg, Bernd, Dr., D-6530 Bingen (DE); Herschel, Michael, Dr., D-6500 Mainz (DE); Rohr, Uwe, Dr., D-6535 Gau-Algesheim (DE)

(56) Entgegenhaltungen:
- EP-A- 0 186 087
- EP-A- 0 227 988
- EP-A- 0 282 971

## Beschreibung

Die vorliegende Erfindung betrifft die transdermale Applikation von 2-Amino-6-n-propylamino-4,5,6,7-tetrahydrobenzothiazol, insbesondere seines (-)-Enantiomeren sowie deren pharmakologisch verträglichen Säureadditionssalze.

Die genannten Verbindungen sind als Arzneistoffe bekannt, wobei die Behandlung der Schizophrenie und die Behandlung des Parkinsonismus bzw. der parkinsonschen Krankheit im Vordergrund stehen. Einzelheiten zur Herstellung dieser Verbindungen können der europäischen Patentanmeldung, EP-A- 0 186 087 entnommen werden. Es hat sich nunmehr gezeigt, daß übliche galenische Zübereitungen wie sie bisher vorgeschlagen wurden, nicht dazu geeignet sind, den Wirkstoff in zufriedenstellender Weise beim Patienten verfügbar zu machen. Bereits bei einer Dosierung von 100 bis 200 »g/Tag wurden orthostatische Nebenwirkungen beim Patienten beobachtet. Aufgrund bisheriger Befunde sind zur Erzielung einer pharmakologischen Wirkung Einzeldosen zwischen 50 und 300 »g notwendig, wobei die gesamte Tagesdosis entsprechend höher liegt.

Es ist die Aufgabe der vorliegenden Erfindung ein Arzneimittel zur Verfügung zu stellen, mit dem 2-Amino-6-n-propylamino-4,5,6,7-tetrahydrobenzothiazol wie auch sein (-)-Enantiomeres in Dosierungen > 200 »g/Tag appliziert werden können, ohne daß orthostatische Nebenwirkungen beim Patienten auftreten.

Überraschenderweise wird die gestellte Aufgabe durch eine transdermale Applikation des Wirkstoffes gelöst. Es wurde gezeigt, daß bei einer transdermalen Applikation von Pramipexol (SND 919, Pramipexol) 2 mg/Tag verabreicht werden konnten, ohne daß orthostatische Nebenwirkungen beim Patienten aufgetreten sind. Dies entspricht der 10-fachen Menge, die üblicherweise bei einer oralen Anwendung der Substanz (200 »g/Tag) ohne orthostatische Nebenwirkungen verabreicht werden kann.
Ein weiterer Gegenstand der vorliegenden Erfindung sind transdermale therapeutische Systeme, die als Wirkstoff 2-Amino-6-n-propylamino-4,5,6,7-tetrahydrobenzothiazol oder sein (-)-Enantiomer (SND 919, Pramipexol) enthalten. Die Art und der Aufbau des transdermalen therapeutischen Systems sind nicht als kritisch anzusehen, solange die verwendeten Hilfs- und Trägerstoffe mit den erfindungsgemäß beschriebenen Wirkstoffen verträglich sind und eine zur Erzielung des gewünschten therapeutischen Effekts ausreichende Wirkstofffreigabe erfolgt.

Für eine transdermale Applikation geeignete Systeme sind aus dem Stand der Technik bekannt. So offenbart beispielsweise die US-PS 3 558 122 ein transdermal therapeutisches System, das aus einer wirkstoffundurchlässigen Rückschicht, einem Wirkstoffreservoir und Mittel zur Befestigung des Systems auf der Haut enthält. Dieses System kann spezielle Vorrichtungen - z.B. eine Membrane - enthalten, die die Wirkstofffreigabe steuern. Aus der europäischen Patentschrift 86 997 ist ein Herstellungsverfahren für eine transdermales System in Form eines Polyacrylatfilmes bekannt.
In einer weiteren erfindungsgemäßen Ausführungsform kann der Wirkstoff auch mit Hilfe eines iontophoretischen Systems appliziert werden. Solche Systeme sind beispielsweise in den europäischen Patentanmeldungen 60 452, 178 601, 147 524, 182 765 wie auch in der deutschen Offenlegungsschrift 32 25 748 beschrieben.
Auch wenn die Lösung der gestellten Aufgabe nicht an die Verwendung eines bestimmten Systems gebunden ist - sofern sichergestellt ist, daß dieses eine ausreichende Wirkstofffreigabe aufweist - so werden erfindungsgemäß solche Systeme bevorzugt, die ein Wirkstoffreservoir aus einem emulsionspolymerisierten Polyacrylat aufweisen. Solche Systeme sind in den europäischen Patentschriften 20 905, 86 997 sowie der europäischen Patentanmeldung 209 121 bekannt. Unter Anwendung der in den genannten Patenten beschriebenen Systeme ist es möglich 2-Amino-6-n-propylamino-4,5,6,7-tetrahydrobenzothaizol bzw. seine (-)-Enantiomere (SND 919) in einer Dosis von 0,5 bis 5 mg/Tag zu applizieren, ohne daß orthostatische Nebeneffekte beobachtet werden.

In einer bevorzugten Ausführungsform besteht das erfindungsgemäße System aus einer wirkstoffundurchlässigen Rückschicht, die gleichzeitig als Deckpflaster ausgebildet ist, um das System auf der Haut zu befestigen, einem wirkstoffhaltigen Reservoir und einer abziehbaren Schutzfolie, die das System vor der eigentlichen Anwendung schützt. Bevorzugtes Trägermaterial für den Wirkstoff ist ein emulsionspolymerisiertes Polyacrylat des Typs Eudragit NE 30 D^{R} der Firma Röhm GmbH Darmstadt. Der Wirkstoffanteil in diesem Reservoir beträgt zwischen 5 und 30 Gew. -%, ein bevorzugter Bereich liegt zwischen 7 und 15 Gew.-%.

Die folgenden Beispiele erläutern die Erfindung in nicht beschränkender Weise.

Für die Beispiele wurde als Polyacrylat Eudragit E 30 D der Firma Röhm, Darmstadt eingesetzt.

### Beispiel 1: Wirkstoff Pramipexol (SND 919)

a) Lösungsmittel Aceton
   In einem geeigneten Gefäß werden 8 g gefriergetrocknetes Polyacrylat, 2 g Pramipexol und 91 g Aceton gegeben und etwa 12 Stunden mit einem Magnetrührer gerührt, bis eine homogene, relativ viskose Lösung resultiert. Diese Lösung wird anschließend in Gießformen gegeben, wobei 190 mg/cm² aufgetragen werden. Bei Zimmertemperatur ist das Lösungsmittel Aceton nach etwa 6 Stunden verdampft. Man erhält einen glatten Film, der pro cm² 8,4 mg Polyacrylat und 2 mg Pramipexol aufweist.
b) Lösungsmittel Acetessigester
   Die Ausgangslösung setzt sich wie folgt zusammen: 6 g Polyacrylat, 1,06 Pramipexol und 93,5 g Acetessigester. Bei Auftragung von 390 mg/cm² führt dies zu einem Film mit 2 mg Pramipexol und 11,6 mg Polyacrylat pro cm².
c) Lösungsmittel Methylenchlorid
   Die Werte der Ausgangslösung: 3 g Polyacrylat, 0,74 Pramipexol und 96,6 g Methylenchlorid. Für eine Auftragungsmenge von 650 mg/cm² erhält man nach der Trocknung einen Film mit 2 mg Pramipexol und 9,64 mg Polyacrylat pro cm².

In Analogie zu dem oben beschriebenen Verfahren wurde folgendes Pflaster hergestellt:

| Reservoir: | |
|---|---|
| Material | Eudragit NE 30 D^{R} |
| Fläche | 20 cm² |
| Dicke | 200 »m |
| Wirkstoffgehalt | 9 Gew. % = 43,3 mg SND 919 |

In vivo Freigaberaten von SND 919:
Oben beschriebenes Pflaster wurde zwei Probanden für 3 bzw. 4 Tagen unter medizinisch reproduzierbaren Bedingungen auf dem Arm befestigt. Die applizierte Dosis betrug bei beiden ca. 2,5 mg/Tag. Die gemessenen Blutspiegel sind in Tabelle A wiedergegeben. Die Bestimmung erfolgte nach dem Radio Immune Assay (RIA)

**Tabelle A**

| Konzentration von SND 919 im Blut nach Anwendung eines mit 9.0 Gew.-% SND 919 beladenen CPA-Pflasters: Größe 20 cm² | | | |
|---|---|---|---|
| | Tag | Zeit | SND 919 ng/ml |
| Proband A | 3. Tag | 8 : 30 | 0.57 |
| | 4. Tag | 8 : 30 | 1 : 54 |
| Proband B | 3.Tag | 8 : 30 | 0.88 |
| | 3. Tag | 12 : 00 | 1.48 |
| | 3. Tag | 15 : 00 | 1.31 |

In Abb. I ist die in vitro Freigabe von SND 919 aus einem Eudragit NE 30 D® Polyacrylat-Pflaster dargestellt.

| | |
|---|---|
| Pflasterfläche | 20 cm² |
| Wirkstoffmenge | 43,3 mg = 9 % |
| Dicke | 200 »m |

Die Untersuchungen wurden gemäß dem USP 21, achte Ausgabe durchgeführt.

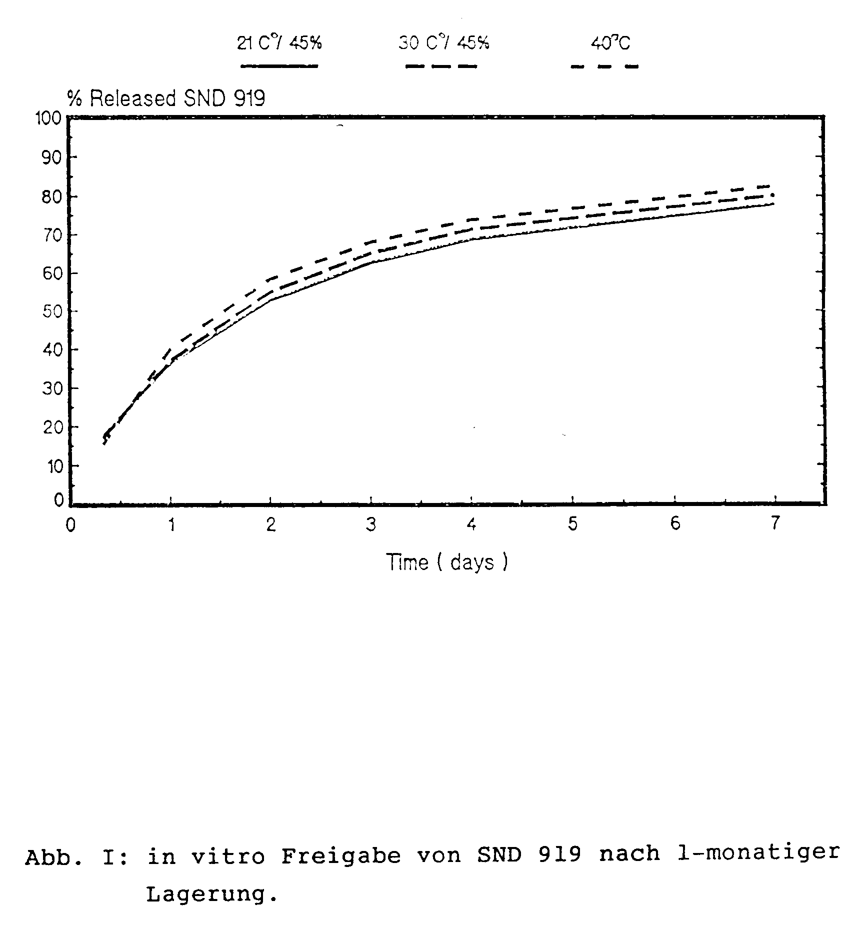

## Patentansprüche

1. Transdermal therapeutisches System enthaltend eine wirkstoffundurchlässige Rückschicht, ein Wirkstoffreservoir und Mittel zur Befestigung auf der Haut, dadurch gekennzeichnet, daß das Wirkstoffreservoir 2-Amino-6-n-propylamino-4,5,6,7-tetrahydrobenzothiazol oder sein (-)-Enantiomeres (SND 919, Pramipexol) enthält.

2. Transdermal therapeutisches System gemäß Anspruch 1, dadurch gekennzeichnet, daß das Wirkstoffreservoir ein emulsionspolymerisiertes Polyacrylat als Wirkstoffträger enthält.

3. Verwendung von 2-Amino-6-n-propylamino-4,5,6,7-tetrahydrobenzothiazol sowie seinem (-)-Entantiomeren zur Herstellung eines Arzneimittels zur transdermalen Behandlung der Schizophrenie.

4. Verwendung von 2-Amino-6-n-propylamino-4,5,6,7-tetrahydrobenzothiazol sowie seinem (-)-Entantiomeren zur Herstellung eines Arzneimittels zur transdermalen zur Behandlung des Parkinsonismus.

5. Verwendung gemäß Anspruch 3 oder 4, dadurch gekennzeichnet, daß das Arzneimittel geeignet ist, 0,5 bis 5 mg des Wirkstoffes pro Tag zu applizieren.

6. Verwendung von 2-Amino-6-n-propylamino-4,5,6,7-tetrahydrobenzothiazol sowie seinem (-)-Entantiomeren zur Herstellung eines transdermalen therapeutischen Systems.

## Claims

1. Transdermally therapeutic system containing a backing layer impervious to the active substance, an active substance reservoir and means for fixing to the skin, characterised in that the active substance reservoir contains 2-amino-6-n-propylamino-4,5,6,7-tetrahydrobenzothiazole or the (-)-enantiomer thereof (SND 919, Pramipexol).

2. Transdermally therapeutic system according to claim 1, characterised in that the active substance reservoir contains an emulsion-polymerised polyacrylate as the active substance carrier.

3. Use of 2-amino-6-n-propylamino-4,5,6,7-tetrahydrobenzothiazole and the (-)-enantiomer thereof for preparing a pharmaceutical composition for the transdermal treatment of schizophrenia.

4. Use of 2-amino-6-n-propylamino-4,5,6,7-tetrahydrobenzothiazole and the (-)-enantiomer thereof for preparing a pharmaceutical composition for the transdermal treatment of Parkinsonism.

5. Use according to claim 3 or 4, characterised in that the pharmaceutical composition is adapted to administer 0.5 to 5 mg of the active substance per day.

6. Use of 2-amino-6-n-propylamino-4,5,6,7-tetrahydrobenzothiazole and the (-)-enantiomer thereof for preparing a transdermal therapeutic system.

## Revendications

1. Système thérapeutique transdermique contenant une couche dorsale imperméable au principe actif, un réservoir à principe actif et des dispositifs pour la fixation sur la peau, caractérisé en ce que le réservoir à principe actif contient du 2-amino-6-n-propylamino-4,5,6,7-tétrahydrobenzothiazole ou son énantiomère (-) (SND 919, Pramipexol).

2. Système thérapeutique transdermique selon la revendication 1, caractérisé en ce que le réservoir à principe actif contient comme support de principe actif un polyacrylate préparé par polymérisation en émulsion.

3. Utilisation du 2-amino-6-n-propylamino-4,5,6,7-tétrahydrobenzothiazole et de son énantiomère (-) pour la préparation d'un médicament pour le traitement transdermique de la schizophrénie.

4. Utilisation du 2-amino-6-n-propylamino-4,5,6,7-tétrahydrobenzothiazole et de son énantiomère (-) pour la préparation d'un médicament pour le traitement transdermique du parkinsonisme.

5. Utilisation selon la revendication 3 ou 4, caractérisée en ce que le médicament convient pour l'application de 0,5 à 5 mg de principe actif par jour.

6. Utilisation du 2-amino-6-n-propylamino-4,5,6,7-tétrahydrobenzothiazole et de son énantiomère (-) pour la préparation d'un système thérapeutique transdermique.
